# DEMANDE DE BREVET EUROPEEN

(11) **EP 4 803 900 A1**
(43) Date de publication de la demande: **09.09.2026**
(21) Numéro de dépôt: 26160468.0
(22) Date de dépôt: 24.02.2026
(51) Int. Cl.: G01N 33/483

(54) **DISPOSITIF UTILISÉ POUR LA MESURE DE PARAMÈTRES EN VUE DE LA SURVEILLANCE D'UN OBJET BIOLOGIQUE**

(30) Priorité: 03.03.2025 FR 2502119
(71) Demandeur: Commissariat à l'Energie Atomique et aux Energies Alternatives, 75015 Paris (FR)
(72) Inventeur: STEGER-POLT, Marie-Hélène, 38054 Grenoble cedex 09 (FR); THOMAS, Yohann, 38054 Grenoble cedex 09 (FR); PHAM, Pascale, 38054 Grenoble cedex 09 (FR)
(74) Mandataire: INNOV-GROUP

(57) **Abrégé**

L'invention concerne un dispositif utilisé pour la mesure de paramètres en vue de la surveillance d'un objet biologique (O) comprenant :
- Un composant (1) comprenant un corps (10) dans lequel est réalisée une chambre (100) comportant un emplacement (2) destiné à recevoir l'objet biologique,
- Une première électrode (E1) et une deuxième électrode (E2) agencées respectivement suivant un premier plan dit supérieur et un deuxième plan dit inférieur, parallèles entre eux, de part et d'autre dudit emplacement (2),
- La première électrode (E1) et la deuxième électrode (E2) étant destinées à être connectées à une source électrique (S), ladite source électrique étant apte à générer une différence de potentiel électrique entre la première électrode et la deuxième électrode,
- La première électrode (E1) et la deuxième électrode (E2) étant positionnées de manière décalée l'une par rapport à l'autre par rapport à un axe transversal, dit axe de lecture (A), passant à travers ledit emplacement (2).

## Description

### Domaine technique de l'invention

L'invention concerne un dispositif utilisé pour la mesure de paramètres en vue de la surveillance d'un objet biologique, via des mesures électriques et éventuellement optiques.

### Etat de la technique

Les organes sur puce représentent une avancée significative en tant qu'outils innovants capables de simuler, même de façon incomplète, la fonctionnalité des organes humains, de manière plus réaliste que les approches traditionnelles, qu'elles soient in vitro ou in vivo sur des animaux.

Par ailleurs, pour suivre en temps réel la viabilité cellulaire ainsi qu'évaluer l'effet de médicaments et de contraintes physiques directement sur ces cellules, l'intégration de capteurs de mesure d'impédance s'avère être une solution prometteuse.

Pour mieux comprendre les cellules et leur organisation en tissus, la mesure de la résistance électrique transépithéliale ou transendothéliale (TEER - Transepithelial-Transendothelial Electrical Resistance) est une technique connue de l'état de l'art. Pour cela, un courant électrique alternatif (AC - Alternate Current) est appliqué au système à une mono fréquence. La spectroscopie d'impédance électrique (EIS - Electrochemical Impedance Spectroscopy) offre aussi un autre outil pour obtenir les valeurs de TEER mais en donnent des informations plus détaillées sur la viabilité et l'intégrité cellulaire puisque qu'un balayage sur une large gamme de fréquences est utilisé.

Des dispositifs de mesure d'une activité électrique ont déjà été décrits dans l'état de la technique. C'est le cas par exemple de la solution décrite dans le brevet FR3114253B1**.** Ce brevet ne s'intéresse cependant pas à l'optimisation des mesures électriques et optiques à travers un objet biologique.

La publication référencée « *van der Helm, M. W. et al. Non-invasive sensing of transepithelial barrier function and tissue differentiation in organs-on-chips using impedance spectroscopy* - *Lab Chip* 19, *452-463 (2019). https:*//*doi.org*/*10.1039*/*C8LC00129D »* décrit un dispositif de mesure de l'activité électrique utilisant quatre ou six électrodes. Le dispositif présenté n'est cependant pas abouti car il ne permet pas de venir corréler les données de mesure d'impédance électrique avec des données de mesure optique, étant donné que la zone qui est optiquement disponible n'est pas équivalente à la zone qui contribue le plus au signal d'impédance. Autrement dit, ce dispositif ne permet pas d'obtenir des données de mesure électrique et des données de mesure optique corrélables entre elles pour une même zone de l'objet biologique.

La demande de brevet US2022/187276A1 décrit un dispositif de mesure de l'activité électrique au travers de cellules biologiques.

Le but de l'invention est donc de proposer un dispositif adapté à la mesure de paramètres en vue de la surveillance d'un objet biologique, dont l'architecture permet de réaliser des mesures électriques et des mesures optiques concentrées sur une même zone de l'objet biologique, permettant ainsi leur corrélation, et donc d'améliorer l'analyse.

### Exposé de l'invention

Ce but est atteint par un dispositif utilisé pour la mesure de paramètres en vue de la surveillance d'un objet biologique comprenant :
- Un composant comprenant un corps dans lequel est réalisée une chambre comportant un emplacement destiné à recevoir l'objet biologique,
- Une première électrode et une deuxième électrode agencées respectivement suivant un premier plan dit supérieur et un deuxième plan dit inférieur, parallèles entre eux, de part et d'autre dudit emplacement,
- La première électrode et la deuxième électrode étant destinées à être connectées à une source électrique, ladite source électrique étant apte à générer une différence de potentiel électrique entre la première électrode et la deuxième électrode,
- La première électrode et la deuxième électrode étant positionnées de manière décalée l'une par rapport à l'autre par rapport à un axe transversal, dit axe de lecture, passant à travers ledit emplacement,
- La première électrode étant de type planaire et s'étendant dans le plan supérieur et réalisée suivant un premier contour fermé comprenant une première portion courbée vers l'intérieur pour former une première concavité,
- La deuxième électrode étant de type planaire et s'étendant dans le plan inférieur et réalisée suivant un deuxième contour fermé comprenant une deuxième portion courbée vers l'intérieur pour former une deuxième concavité,
- La première concavité et la deuxième concavité délimitent entre elles un passage transversal sans électrode, à travers ledit emplacement.

Selon une réalisation particulière, la première électrode et la deuxième électrode sont identiques et la première électrode et la deuxième électrode sont positionnées de manière symétrique par rapport à un point positionné au centre dudit emplacement destiné à recevoir l'objet biologique.

Selon une autre réalisation particulière, la première électrode et la deuxième électrode se présentent chacune sous la forme d'une portion annulaire, réalisée sur une plage angulaire comprise entre 120° et 180°.

Selon une autre réalisation particulière, la portion annulaire formant respectivement la première électrode et la deuxième électrode comporte une largeur comprise entre 1mm et 4mm.

Selon une autre réalisation particulière, la première électrode et la deuxième électrode comportent chacune une forme de rectangle creusée suivant un côté pour former respectivement la première concavité et la deuxième concavité.

Selon une autre réalisation particulière, chaque électrode est réalisée dans un matériau choisi parmi :
- Un matériau à base de carbone : matériau issu du Graphène (Oxyde de Graphène ou oxyde de graphène réduit : GO ou rGO), matériau de type diamant (DLC ou BDD),
- Métal biocompatible type platine, or, oxyde d'iridium,
- Un matériau transparent et conducteur : Oxyde d'Indium-Etain (ITO), Oxyde de Zinc (ZnO),
- Un mélange des composés précédents.

Selon une autre réalisation particulière, le corps du composant est réalisé dans un matériau choisi parmi le COC, le PMMA, le polycarbonate, et le verre.

L'invention concerne également un système de surveillance d'un objet biologique comprenant une source électrique et un dispositif destiné à recevoir ledit objet biologique, caractérisé en ce que :
- Ledit dispositif est tel que défini ci-dessus, la première électrode et la deuxième électrode étant connectées chacune à une borne distincte de la source électrique,
- Le système comporte des moyens de mesure d'une grandeur électrique lors de l'application d'un signal électrique entre la première électrode et la deuxième électrode,
- Le système comporte une unité de traitement à laquelle sont connectés les moyens de mesure de la grandeur électrique, configurée pour traiter des données de mesure issues desdits moyens de mesure de la grandeur électrique.

Selon une particularité, le système comporte des moyens de suivi optique comportant au moins une source lumineuse positionnée pour émettre suivant ledit axe de lecture et un capteur positionné en vis-à-vis de ladite source lumineuse, à l'opposé de celle-ci par rapport audit emplacement, ledit capteur étant connecté à ladite unité de traitement, l'unité de traitement étant configurée pour traiter des données de mesure optique issues dudit capteur.

Selon une autre particularité, la grandeur électrique est l'impédance électrique.

### Brève description des figures

D'autres caractéristiques et avantages vont apparaître dans la description détaillée qui suit faite en regard des dessins annexés dans lesquels :
- La figure 1 représente, en vue de côté, un système de surveillance d'un objet biologique, conforme à l'invention ;
- La figure 2 montre plusieurs configurations d'agencement des deux électrodes ; Sur chaque configuration, les électrodes sont montrées en vue de dessus, mais il faut comprendre qu'elles sont agencées selon deux plans distincts (superposées suivant Z) ;
- Les figures 3A et 3B montrent deux diagrammes illustrant la pertinence de l'invention ;

### Description détaillée d'au moins un mode de réalisation

Dans la suite de la description, on utilise un repère orthonormé X, Y, Z.

Dans la suite de la description, les termes "inférieur", "supérieur", "au-dessus", "au-dessous" ou équivalent sont à considérer en tenant compte de la direction selon l'axe Z.

Le terme "longitudinal" est à comprendre dans les directions parallèles au plan X, Y et le terme "transversal" dans les directions selon Z.

L'invention vise à surveiller un objet biologique O, via des mesures électriques (par exemple d'impédance électrique) à travers cet objet biologique O, et éventuellement via des mesures optiques à travers l'objet biologique O.

L'objet biologique O est par exemple une couche de cellules ensemencée sur une membrane poreuse 103 (voir ci-après) ou un agrégat de cellules. Par agrégat de cellules, on entend, selon l'invention, l'auto-assemblage d'un ou plusieurs types de cellules en trois dimensions. Un tel agrégat de cellules peut notamment s'appeler sphéroïde, organoïde, tumoroïde, neuro-sphère. Cet agrégat peut également être un îlot de Langerhans, un tapis de cellules représentatif d'un modèle de peau ou de poumon. Dans la suite de la description, on utilisera de manière générique le terme "objet biologique", référencé O, pour évoquer un tel agrégat ou une telle couche, ce terme étant classiquement employé dans le domaine de la culture de cellules vivantes. De manière non limitative, un tel objet biologique O peut par exemple présenter un diamètre allant de quelques dizaines de µm à quelques milliers de µm.

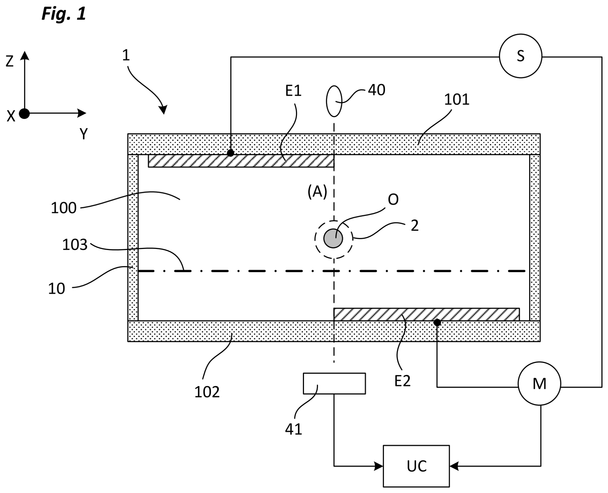

En référence à la figure 1, le dispositif comporte un composant microfluidique 1 comportant un corps 10, dans lequel est réalisée une chambre 100 comportant un emplacement 2 destiné à recevoir l'objet biologique O. Ledit corps 10 présente avantageusement une face supérieure 101 et une face inférieure 102 transparentes aux rayons lumineux employés pour une surveillance optique de l'objet biologique O. Ledit corps peut être réalisé par exemple dans un matériau de type COC, PMMA, PDMS, polycarbonate, verre ou une combinaison de ces matériaux.

La chambre 100 peut avantageusement être divisée en deux espaces superposés selon la direction Z, séparés entre eux par une membrane 103 poreuse agencée dans un plan parallèle à X, Y. Ces deux espaces peuvent être employés pour permettre la co-culture de deux types de cellules, ou pour permettre une perfusion microfluidique de cellules placées dans l'un ou l'autre des deux espaces. L'emplacement 2 pour l'objet biologique O peut être disposé dans l'un ou l'autre des deux espaces ou dans la chambre, si la membrane 103 n'est pas présente. La membrane 103 poreuse peut être également choisie conductrice électrique. Elle peut ainsi être employée pour effectuer des mesures électriques, associée à une autre électrode du dispositif (voir ci-dessous les deux électrodes E1, E2).

Le dispositif comporte également deux électrodes conductrices, dites première électrode E1 et deuxième électrode E2. Ces électrodes sont biocompatibles et sont destinées à être associées entre elles pour effectuer des mesures électriques à travers l'objet biologique O, par exemple des mesures d'impédance électrique. Les deux électrodes E1, E2 sont avantageusement de type planaire (avec une épaisseur selon Z faible au regard des deux autres dimensions suivant X et Y - au moins un facteur dix entre les dimensions).

Les deux électrodes E1, E2 sont par exemple fabriquées dans :
- Un matériau à base de carbone : matériau issu du Graphène (Oxyde de Graphène ou oxyde de graphène réduit : GO ou rGO), matériau de type diamant (DLC ou BDD),
- Métal biocompatible type platine, or, oxyde d'iridium,
- Un matériau transparent et conducteur : Oxyde d'Indium-Etain (ITO), Oxyde de Zinc (ZnO),
- Un mélange des composés précédents (par exemple graphite + noir de carbone).

La première électrode E1 et la deuxième électrode E2 sont destinées à être connectées à une source électrique S d'un système plus complet, ladite source électrique S étant apte à générer une différence de potentiel électrique entre la première électrode E1 et la deuxième électrode E2.

La première électrode E1 et la deuxième électrode E2 sont agencées respectivement suivant un premier plan dit supérieur et un deuxième plan dit inférieur, distincts, parallèles entre eux, de part et d'autre dudit emplacement 2. Les deux plans d'agencement des électrodes sont arrangés parallèles à X, Y.

Par le terme « électrode », on entend un élément conducteur pouvant être mis à un potentiel électrique particulier. Les deux électrodes E1, E2 sont ainsi formées de deux éléments conducteurs distincts et sont destinées à être placées à des potentiels électriques distincts en étant connectées à la source électrique S.

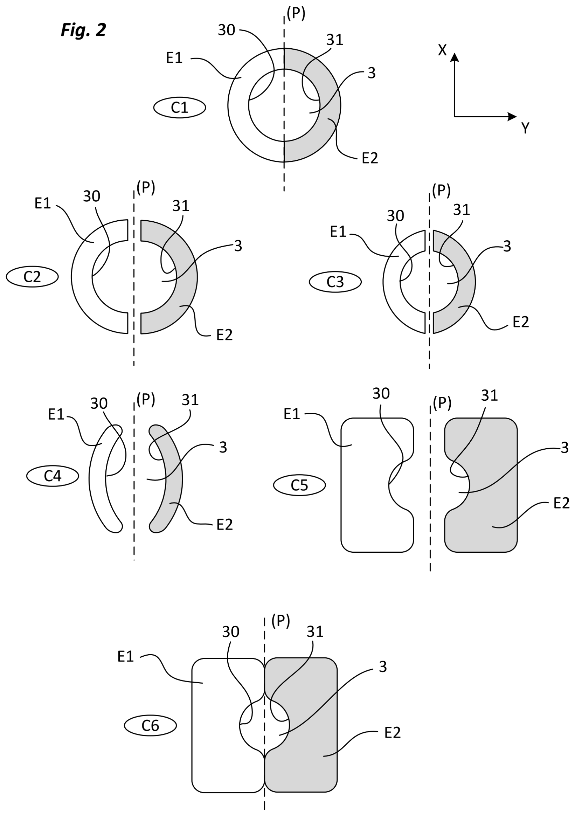

Selon un aspect particulier de l'invention, pour permettre de focaliser les mesures électriques à travers l'emplacement 2 destiné à recevoir l'objet biologique O, le dispositif utilise un agencement particulier des électrodes, et les électrodes possèdent une forme déterminée :
- La première électrode E1 et la deuxième électrode E2 sont en effet positionnées de manière décalée l'une par rapport à l'autre, par rapport à un axe (A) transversal (selon Z) passant par le centre dudit emplacement 2.
- La première électrode E1 de type planaire se présente sous la forme d'une première bande s'étendant dans le plan supérieur et réalisée suivant un premier contour fermé comprenant une première portion courbée vers l'intérieur formant une première concavité 30 (figure 2),
- La deuxième électrode E2 de type planaire se présente sous la forme d'une deuxième bande s'étendant dans le plan inférieur et réalisée suivant un deuxième contour fermé comprenant une deuxième portion courbée vers l'intérieur formant une deuxième concavité 31 (figure 2),
- La première concavité 30 et la deuxième concavité 31 délimitent entre elles un passage transversal (référencé 3 sur la figure 2) sans électrode, à travers ledit emplacement 2.

Cette forme particulière des deux électrodes E1, E2 permet de garantir l'application homogène d'un signal électrique à travers ledit emplacement 2 destiné à recevoir l'objet biologique O. De plus, la présence du passage transversal 3 délimité par les deux concavités 30, 31 permet de créer un accès axial pour une surveillance optique de l'objet biologique O présent audit emplacement 2.

Le dispositif de l'invention est ainsi adapté pour permettre une corrélation entre mesures électriques et mesures optiques à travers l'objet biologique O.

La figure 2 présente plusieurs configurations possibles d'électrodes, adaptées pour remplir les deux objectifs précités.

Dans les configurations proposées, les deux électrodes E1, E2 sont positionnées de manière symétrique par rapport à un point qui est positionné avantageusement au centre de l'emplacement 2 réservé à l'objet biologique O. Sur la figure 2, les électrodes E1, E2 sont montrées en vue de dessus et ne sont pas dans un même plan.

Figure 2 - C1 : Une première configuration consiste à réaliser chaque électrode E1, E2 sous la forme d'une portion d'anneau, s'étendant par exemple sur une plage angulaire de 180°.

Tout en conservant la symétrie, il serait possible de former la portion annulaire sur une plage angulaire inférieure à 180°, par exemple comprise entre 120° et 180°.

La largeur (dans le plan parallèle à X, Y) de la bande formant chaque électrode est avantageusement constante et est par exemple comprise entre 200µmm et 4mm. Dans cette configuration C1, les deux électrodes E1, E2 sont chacune tangentes par leurs deux extrémités à un même plan vertical (selon Z) (dit plan vertical axial (P)) passant par le centre de symétrie.

Le creux formé par la courbure de l'électrode permet de créer sa concavité, nécessaire pour former le passage transversal.

Des simulations concluantes ont été réalisées avec cette configuration C1, avec un diamètre externe de 10mm, un diamètre interne de 2mm ou un diamètre interne de 3mm (voir tableau ci-dessous).

Figure 2 - C2 : Dans cette configuration, les deux électrodes E1, E2 sont identiques à celles de la configuration C1 mais sont écartées l'une de l'autre par rapport au plan vertical axial (P).

Des simulations concluantes ont été réalisées avec cette configuration C2, avec un diamètre externe de 10mm, un diamètre interne de 2mm, les deux électrodes étant écartées de 2mm.

Figure 2 - C3 : Dans cette configuration, les deux électrodes E1, E2 sont réalisées avec une courbure inférieure à celle proposée dans les deux configurations précédentes. Comme sur la configuration C2, les deux électrodes E1, E2 sont également décalées dans leur plan respectif d'une distance donnée par rapport au plan vertical axial (P).

Figure 2 - C4 : Dans cette configuration, les deux électrodes E1, E2 ont leurs deux extrémités libres arrondies, alors qu'elles étaient à bords droits dans les configurations précédentes. On évite ainsi les points de concentration de courant dans des zones non pertinentes. Cette configuration montre également deux électrodes avec une courbure supérieure à celle des deux premières configurations.

Figure 2 - C5 et figure 2 - C6 : Dans cette configuration, chaque électrode E1, E2 présente une forme générale rectangulaire, creusée sur un seul côté pour former sa concavité 30, 31 respective. Les deux électrodes sont par exemple écartées l'une de l'autre par rapport au plan vertical axial (configuration C5) ou chacune tangente à ce plan (configuration C6). Les deux concavités 30, 31 en opposition permettent de créer le passage transversal 3, utile pour réaliser un suivi optique de l'objet biologique O lorsque celui-ci est dans l'emplacement 2 prévu de la chambre 100.

Dans tous les cas, une optimisation de la configuration pourra s'avérer nécessaire, même si quelques règles de généralisation peuvent être dressées, notamment pour les configurations C1 à C4 :
- Diamètre externe compris entre 8mm et 15mm, avantageusement compris entre 10 mm et 15mm ;
- Diamètre interne qui dépend du diamètre externe, cependant la différence (la largeur de la bande) doit être supérieure à 1mm minimum ;

Pour les configurations C1 à C4, il faut également noter que la présence d'un grand diamètre interne équivaut à une faible largeur de demi-cercle, ce qui peut entraîner une diminution de la densité de courant maximale. Cela s'explique également par le fait qu'à plus faible surface, le système en devient plus résistif. Cet aspect peut s'avérer gênant si le système est utilisé à basse fréquence (par exemple entre 10Hz et 100 Hz). On assiste également à un aplatissement des densités de courant maximales. Cet aspect est plutôt avantageux pour l'invention puisqu'une plus grande zone d'analyse aura la même densité.

Pour les configurations C1 à C4, on obtient ainsi les résultats suivants :

| Configuration | Courant maximal à 100Hz (en mA/m2) |
|---|---|
| C1 (2 mm) | 1510.2 |
| C1 (3 mm) | 1510.2 |
| C2 | 1408.0 |
| C3+C4 | 716.9 |

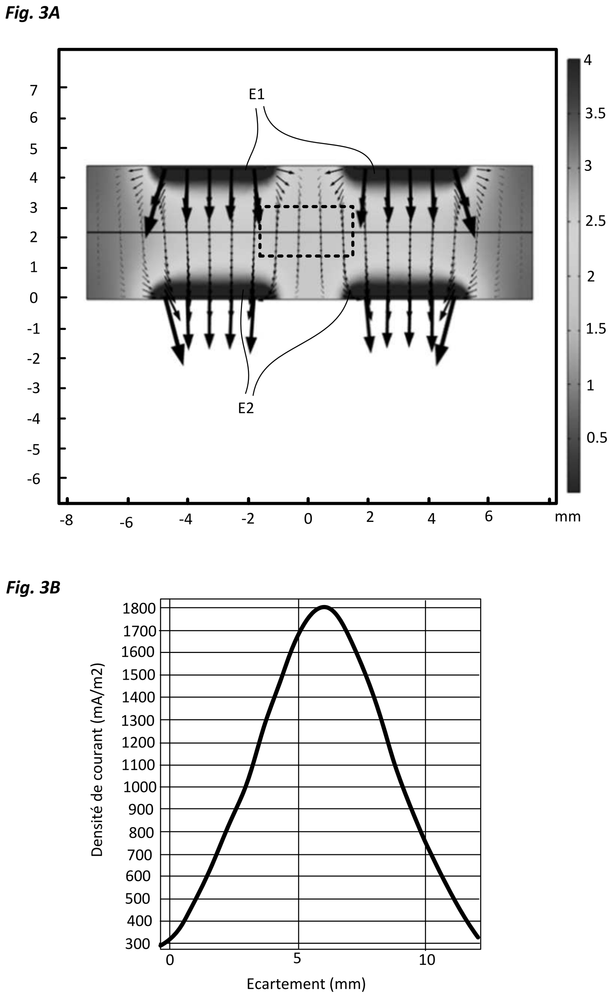

A l'aide des configurations décrites ci-dessus, les diagrammes de la figure 3A et de la figure 3B permettent de constater que la densité de courant atteint un maximum dans la zone définie par ledit emplacement 2. Ces diagrammes ont été établis avec la configuration C1 décrite ci-dessus.

La figure 3A illustre, par un diagramme de chaleur, la densité de courant entre les deux électrodes E1, E2. Sur cette figure 3A, le composant est montré de sorte à voir les deux électrodes E1, E2 superposées, dans le plan de coupe transversal axial.

Sur cette figure 3A, dans la zone marquée par un carré en pointillés correspondant audit emplacement 2, on remarque que la densité de courant est homogène (couleur grise homogène), avec des lignes de courant globalement parallèles.

Ceci est confirmé par le diagramme de la figure 3B qui montre en abscisses l'écartement maximal selon Y (en mm) entre les deux électrodes E1, E2 et en ordonnées, la densité de courant (en mA/m²). Au niveau de la zone centrale (qui correspond à l'emplacement 2), la densité de courant est maximale et fait un pic.

Grâce à l'agencement de l'invention, on est ainsi capable de concentrer le courant à travers l'objet biologique O et donc de fiabiliser les mesures. De plus, les configurations présentées offrent chacune la possibilité de réaliser, en plus, une surveillance optique axiale de l'objet biologique O.

Le dispositif tel que décrit ci-dessus est employé dans un système de surveillance d'un objet biologique. En référence à la figure 1, ce système de surveillance comporte la source électrique S précitée, dont les deux bornes viennent se connecter électriquement respectivement à la première électrode E1 et à la deuxième électrode E2. Cette source électrique est commandée pour appliquer un signal alternatif de tension de faible amplitude entre les deux électrodes, lorsque l'objet biologique O est positionné dans ledit emplacement 2.

Le système comporte des moyens de mesure M d'une grandeur électrique (par exemple un courant électrique) entre les deux électrodes E1, E2, lors de l'application de la différence de potentiel. Cette grandeur électrique est par exemple une impédance électrique. Grâce à ces mesures, on vient ainsi caractériser l'objet biologique O, et suivre son évolution, par exemple lors d'une étape de culture.

En outre, de manière avantageuse, le système peut comporter des moyens de suivi optique, utilisés pour exploiter la présence du passage transversal défini ci-dessus. Ces moyens de suivi optique peuvent comporter une source lumineuse 40 positionnée pour émettre un signal lumineux suivant un axe, dit axe de lecture, passant par le centre dudit emplacement 2 destiné à recevoir l'objet biologique O. Les moyens de suivi optique comportent également un capteur 41, positionné de manière à récupérer les signaux lumineux, après passage à travers l'objet biologique. Ce capteur 41 est positionné dans l'axe de lecture optique, à l'opposé de la source par rapport audit emplacement. Il serait également possible d'avoir deux capteurs, l'un au-dessus et l'un au-dessous pour mieux surveiller l'objet biologique O.

Le système comporte une unité de traitement UC à laquelle sont connectés les moyens de mesure M de la grandeur électrique et le capteur 41 des moyens de suivi optique. L'unité de traitement UC est chargée de recueillir les données de mesure issues des moyens de mesure de la grandeur électrique et du capteur 41 des moyens de suivi optique. Elle est configurée pour effectuer une corrélation entre les deux types de données, pour améliorer la surveillance et le suivi de croissance d'un objet biologique O et ainsi savoir comment évolue une culture de cellules une fois qu'elle est établie, par exemple en réponse à un stress, un médicament, une substance toxique, lors d'une culture.

## Revendications

1. Dispositif utilisé pour la mesure de paramètres en vue de la surveillance d'un objet biologique (O) comprenant :
- Un composant (1) comprenant un corps (10) dans lequel est réalisée une chambre (100) comportant un emplacement (2) destiné à recevoir l'objet biologique,
- Une première électrode (E1) et une deuxième électrode (E2) agencées respectivement suivant un premier plan dit supérieur et un deuxième plan dit inférieur, parallèles entre eux, de part et d'autre dudit emplacement (2),
- La première électrode (E1) et la deuxième électrode (E2) étant destinées à être connectées à une source électrique (S), ladite source électrique étant apte à générer une différence de potentiel électrique entre la première électrode et la deuxième électrode,
- La première électrode (E1) et la deuxième électrode (E2) étant positionnées de manière décalée l'une par rapport à l'autre par rapport à un axe transversal, dit axe de lecture (A), passant à travers ledit emplacement (2),
- **Caractérisé en ce que** :
- La première électrode (E1) est de type planaire et s'étend dans le plan supérieur et réalisée suivant un premier contour fermé comprenant une première portion courbée vers l'intérieur formant une première concavité (30),
- La deuxième électrode (E2) est de type planaire et s'étend dans le plan inférieur et réalisée suivant un deuxième contour fermé comprenant une deuxième portion courbée vers l'intérieur formant une deuxième concavité (31),
- La première concavité (30) et la deuxième concavité (31) délimitent entre elles un passage transversal (3) sans électrode, à travers ledit emplacement (2).

2. Dispositif selon la revendication 1, **caractérisé en ce que** la première électrode (E1) et la deuxième électrode (E2) sont identiques et **en ce que** la première électrode (E1) et la deuxième électrode (E2) sont positionnées de manière symétrique par rapport à un point positionné au centre dudit emplacement (2) destiné à recevoir l'objet biologique.

3. Dispositif selon la revendication 2, **caractérisé en ce que** la première électrode (E1) et la deuxième électrode (E2) se présentent chacune sous la forme d'une portion annulaire, réalisée sur une plage angulaire comprise entre 120° et 180°.

4. Dispositif selon la revendication 3, **caractérisé en ce que** la portion annulaire formant respectivement la première électrode (E1) et la deuxième électrode (E2) comporte une largeur comprise entre 1mm et 4mm.

5. Dispositif selon la revendication 2, **caractérisé en ce que** la première électrode (E1) et la deuxième électrode (E2) comportent chacune une forme de rectangle creusée suivant un côté pour former respectivement la première concavité et la deuxième concavité.

6. Dispositif selon l'une des revendications 1 à 5, **caractérisé en ce que** chaque électrode est réalisée dans un matériau choisi parmi :
- Un matériau à base de carbone,
- Un métal biocompatible,
- Un matériau transparent et conducteur : Oxyde d'Indium-Etain (ITO), Oxyde de Zinc,
- Un mélange des composés précédents.

7. Dispositif selon l'une des revendications 1 à 6, **caractérisé en ce que** le corps du composant est réalisé dans un matériau choisi parmi le COC, le PMMA, le polycarbonate, et le verre.

8. Système de surveillance d'un objet biologique comprenant une source électrique (S) et un dispositif destiné à recevoir ledit objet biologique, **caractérisé en ce que** :
- Ledit dispositif est tel que défini dans l'une des revendications 1 à 5, la première électrode (E1) et la deuxième électrode (E2) sont connectées chacune à une borne distincte de la source électrique (S),
- Le système comporte des moyens de mesure (M) d'une grandeur électrique lors de l'application d'un signal électrique entre la première électrode (E1) et la deuxième électrode (E2),
- Le système comporte une unité de traitement (UC) à laquelle sont connectés les moyens de mesure (M) de la grandeur électrique, configurée pour traiter des données de mesure issues desdits moyens de mesure (M) de la grandeur électrique.

9. Système selon la revendication 8, **caractérisé en ce qu'**il comporte des moyens de suivi optique comportant au moins une source lumineuse (40) positionnée pour émettre suivant ledit axe de lecture (A) et un capteur (41) positionné en vis-à-vis de ladite source lumineuse (40), à l'opposé de celle-ci par rapport audit emplacement (2), ledit capteur (41) étant connecté à ladite unité de traitement (UC), l'unité de traitement étant configurée pour traiter des données de mesure optique issues dudit capteur.

10. Système selon la revendication 8 ou 9, **caractérisé en ce que** la grandeur électrique est l'impédance électrique.
